# EUROPÄISCHE PATENTSCHRIFT

(11) **EP 4 041 075 B1**
(45) Veröffentlichungstag und Bekanntmachung des Hinweises auf die Patenterteilung: **13.08.2025**
(21) Anmeldenummer: 20781546.5
(22) Anmeldetag: 05.10.2020
(51) Int. Cl.: A61B 5/055, A61B 5/00, G01R 33/56

(54) **ERZEUGUNG VON MRT-AUFNAHMEN DER LEBER OHNE KONTRASTVERSTÄRKUNG**
GENERATION OF MRI IMAGES OF THE LIVER WITHOUT CONTRAST ENHANCEMENT
GÉNÉRATION D'ENREGISTREMENTS IRM DU FOIE SANS REHAUSSEMENT DU CONTRASTE

(30) Priorität: 08.10.2019 EP 19201919
(43) Veröffentlichungstag der Anmeldung: 17.08.2022
(73) Patentinhaber: Bayer Aktiengesellschaft, 51373 Leverkusen (DE)
(72) Erfinder: KNOBLOCH, Gesine, 10437 Berlin (DE); LIENERTH, Christian, 60486 Frankfurt/Main (DE)
(74) Vertreter: BIP Patents
(86) Internationale Anmeldenummer: PCT/EP2020/077767
(87) Internationale Veröffentlichungsnummer: WO 2021/069338

(56) Entgegenhaltungen:
- KOICHIRO YASAKA ET AL: "Deep Learning with Convolutional Neural Network for Differentiation of Liver Masses at Dynamic Contrast-enhanced CT: A Preliminary Study", RADIOLOGY, vol. 286, no. 3, 1 March 2018 (2018-03-01), US, pages 887 - 896, XP055634059, ISSN: 0033-8419, DOI: 10.1148/radiol.2017170706
- ENHAO GONG ET AL: "Deep learning enables reduced gadolinium dose for contrast-enhanced brain MRI : Deep Learning Reduces Gadolinium Dose", JOURNAL OF MAGNETIC RESONANCE IMAGING, vol. 48, no. 2, 13 February 2018 (2018-02-13), US, pages 330 - 340, XP055656267, ISSN: 1053-1807, DOI: 10.1002/jmri.25970
- UNKNOWN: "FACHINFORMATION Primovist", 1 May 2016 (2016-05-01), pages 1 - 5, XP055656320, Retrieved from the Internet <URL:https://s3.eu-central-1.amazonaws.com/prod-cerebro-ifap/media_all/71888.pdf> [retrieved on 20200109]

## Beschreibung

Die vorliegende Erfindung befasst sich mit der Erzeugung von künstlichen MRT-Aufnahmen der Leber. Gegenstände der vorliegenden Erfindung sind ein Verfahren, ein System und ein Computerprogrammprodukt zur Erzeugung von MRT-Aufnahmen der Leber ohne Kontrastverstärkung.

Die Magnetresonanztomographie, abgekürzt MRT oder MR (engl. MRI: *Magnetic Resonance Imaging*)*,* ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer T1-Verkürzung führen. Eine Verkürzung der T1-Zeit führt zu einer Zunahme der Signalintensität in T1-gewichteten Sequenzen, eine Verkürzung der T2-Zeit führt zu einer Abnahme der Signalintensität in T2-gewichteten Sequenzen.

Die Wirkung dieser Kontrastmittel ist indirekt, da das Kontrastmittel selbst kein Signal abgibt, sondern nur die Signalintensität der Wasserstoffprotonen in seiner Umgebung beeinflusst.

In T1-gewichteten Aufnahmen führen die paramagnetischen Kontrastmittel zu einer helleren (signalreicheren) Darstellung der Bereiche, die Kontrastmittel beinhalten, gegenüber den Bereichen, die kein Kontrastmittel beinhalten.

In T2-gewichteten Aufnahmen führen superparamagnetisches Kontrastmittel zu einer dunkleren (signalärmeren) Darstellung der Bereiche, die Kontrastmittel beinhalten, gegenüber den Bereichen, die kein Kontrastmittel beinhalten.

Die vorliegende Erfindung befasst sich mit der Erzeugung von künstlichen MRT-Aufnahmen der Leber. Gegenstände der vorliegenden Erfindung sind ein Verfahren, ein System und ein Computerprogrammprodukt zur Erzeugung von MRT-Aufnahmen der Leber ohne Kontrastverstärkung.

Koichiro Yasaka et al., Radiology, Bd. 286, Nr. 3, 1. März 2018, XP055634059 offenbart die Verwendung von Computertomographie (CT) Bilder der Leber für ein Krebsvorhersagemodell, welches mittels überwachten Lernens trainiert worden ist.

Enhao Gong et al., Journal of Magnetic Resonance Imaging, Bd. 48 Nr. 2, 13. Februar 2018, XP055656267 offenbart die Vorhersage von konktrastverstärkten Gehirn Magnetresonanztomographie (MRT) Bilder mittels Gehirn MRT Bilder mit geringer Kontrastverstärkung.

Die Magnetresonanztomographie, abgekürzt MRT oder MR (engl. MRI: *Magnetic Resonance Imaging*)*,* ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer T1-Verkürzung führen. Eine Verkürzung der T1-Zeit führt zu einer Zunahme der Signalintensität in T1-gewichteten Sequenzen, eine Verkürzung der T2-Zeit führt zu einer Abnahme der Signalintensität in T2-gewichteten Sequenzen.

Die Wirkung dieser Kontrastmittel ist indirekt, da das Kontrastmittel selbst kein Signal abgibt, sondern nur die Signalintensität der Wasserstoffprotonen in seiner Umgebung beeinflusst.

In T1-gewichteten Aufnahmen führen die paramagnetischen Kontrastmittel zu einer helleren (signalreicheren) Darstellung der Bereiche, die Kontrastmittel beinhalten, gegenüber den Bereichen, die kein Kontrastmittel beinhalten.

In T2-gewichteten Aufnahmen führen superparamagnetisches Kontrastmittel zu einer dunkleren (signalärmeren) Darstellung der Bereiche, die Kontrastmittel beinhalten, gegenüber den Bereichen, die kein Kontrastmittel beinhalten.

Sowohl eine signalreichere als auch eine signalärmere Darstellung führen zu einer Kontrastverstärkung.

Ein Beispiel für ein superparamagnetisches Kontrastmittel sind Eisenoxidnanopartikel (SPIO, engl.: *superparamagnetic iron oxide*)*.*

Beispiele für paramagnetische Kontrastmittel sind Gadolinium-Chelate wie Gadopentetat-Dimeglumin (Handelsname: Magnevist^{®} u.a.), Gadobenat-Dimeglumin (Handelsname: Multihance^{®}), Gadotersäure (Dotarem^{®}, Dotagita^{®}, Cyclolux^{®}), Gadodiamid (Omniscan^{®}), Gadoteridol (ProHance^{®}) und Gadobutrol (Gadovist^{®}).

Nach ihrem Verteilungsmuster im Gewebe können extrazelluläre, intrazelluläre und intravaskuläre Kontrastmittel unterschieden werden.

Kontrastmittel auf der Basis der Gadoxetsäure zeichnen sich dadurch aus, dass sie spezifisch von Leberzellen, den Hepatozyten, aufgenommen werden, sich im Funktionsgewebe (Parenchym) anreichern und die Kontraste in gesundem Lebergewebe verstärken. Die Zellen von Zysten, Metastasen und der meisten Leberzellkarzinome arbeiten nicht mehr wie normale Leberzellen, nehmen das Kontrastmittel nicht oder kaum auf, werden nicht verstärkt dargestellt und werden damit erkennbar und lokalisierbar.

Beispiele für Kontrastmittel auf der Basis der Gadoxetsäure sind in US 6,039,931A beschrieben; sie sind kommerziell zum Beispiel unter den Markennamen Primovist^{®} oder Eovist^{®} erhältlich.

Der kontrastverstärkende Effekt von Primovist^{®}/Eovist^{®} wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels.

Primovist^{®} kann zur Detektion und Charakterisierung von Tumoren in der Leber eingesetzt werden. Die Blutversorgung des gesunden Lebergewebes erfolgt in erster Linie über die Pfortader (*Vena portae*)*,* während die Leberarterie (*Arteria hepatica*) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Bei der durch Primovist^{®} erzielten Kontrastverstärkung während der Anflutungsphase beobachtet man typische Perfusionsmuster, die Informationen für die Charakterisierung der Läsionen liefern. Die Darstellung des Anreicherungsverhaltens (wash-in), des Auswaschverhaltens (wash-out) sowie der Vaskularisierung hilft, die Läsionstypen zu charakterisieren und den räumlichen Zusammenhang zwischen Tumor und Blutgefäßen zu bestimmen.

Bei T1-gewichteten Bildern führt Primovist^{®} 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als dunklere Bereiche auftreten.

Allerdings treten in der hepatobiliären Phase auch die Blutgefäße als dunkle Bereiche auf, so dass in den MRT-Aufnahmen, die während der hepatobiliären Phase erzeugt werden, eine Differenzierung von Leberläsionen und Blutgefäßen allein anhand des Kontrasts i.d.R. nicht möglich ist. Eine Differenzierung zwischen Leberläsionen und Blutgefäßen kann nur im Zusammenhang mit weiteren MRT-Aufnahmen z.B. der dynamischen Phase (bei der die Blutgefäße hervorgehoben werden) oder auch unter Zuhilfenahme von MRT-Aufnahmen ohne eine durch ein Kontrastmittel herbeigeführte Kontrastverstärkung erfolgen. Wenn aber beispielsweise ein für ein Untersuchungsobjekt verkürztes MRT-Aufnahmeverfahren angewendet wird, z.B. wenn ein Kontrastmittels bereits eine gewisse Zeitspanne vor der MRT-Aufnahme appliziert wird, um direkt MRT-Aufnahmen innerhalb der hepatobiliären Phase aufzunehmen und dann - nach einer zweiten Kontrastmittel-Applikation - MRT-Aufnahmen der dynamischen Phase erstellt werden, kann eine MRT-Aufnahme ohne Kontrastverstärkung (native MRT-Aufnahme) im gleichen MRT-Aufnahmevorgang nicht mehr erstellt werden.

Diesem Problem widmet sich die vorliegende Erfindung mit den Gegenständen der unabhängigen Patentansprüche. Bevorzugte Ausführungsformen der vorliegenden Erfindung finden sich in den abhängigen Patentansprüchen, in dieser Beschreibung und in den Zeichnungen.

Ein erster Gegenstand der vorliegenden Erfindung ist ein Verfahren umfassend die Schritte
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind,
- Empfangen mindestens einer zweiten MRT-Aufnahme desselben Untersuchungsobjekts, wobei die mindestens eine zweite MRT-Aufnahme dieselbe Leber oder denselben Teil der Leber zeigt, wobei gesunde Leberzellen durch ein Kontrastmittel kontrastverstärkt dargestellt sind,
- Zuführen der empfangenen MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen, in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- Empfangen einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, von dem Vorhersagemodell,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

Ein weiterer Gegenstand der vorliegenden Erfindung ist ein System umfassend
- eine Empfangseinheit,
- eine Steuer- und Recheneinheit und
- eine Ausgabeeinheit,
   - wobei die Steuer- und Recheneinheit konfiguriert ist, die Empfangseinheit zu veranlassen, mindestens eine erste MRT-Aufnahme eines Untersuchungsobjekts zu empfangen, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind,
   - wobei die Steuer- und Recheneinheit konfiguriert ist, die Empfangseinheit zu veranlassen, mindestens eine zweite MRT-Aufnahme eines Untersuchungsobjekts zu empfangen, wobei die mindestens eine zweite MRT-Aufnahme dieselbe Leber oder denselben Teil der Leber zeigt, wobei gesunde Leberzellen durch ein Kontrastmittel kontrastverstärkt dargestellt sind,
   - wobei die Steuer- und Recheneinheit konfiguriert ist, anhand der empfangenen MRT-Aufnahmen eine oder mehrere MRT-Aufnahmen vorherzusagen, wobei die eine oder die mehreren vorhergesagten MRT-Aufnahmen die Leber oder einen Teil der Leber des Untersuchungsobjekts ohne eine durch ein Kontrastmittel herbeigeführte Kontrastverstärkung zeigen,
   - wobei die Steuer- und Recheneinheit konfiguriert ist, die Ausgabeeinheit zu veranlassen, die eine oder die mehreren vorhergesagte MRT-Aufnahmen anzuzeigen, auszugeben oder in einem Datenspeicher zu speichern.

Ein weiterer Gegenstand der vorliegenden Erfindung ist ein Computerprogrammprodukt umfassend ein Computerprogramm, das in einen Arbeitsspeicher eines Computers geladen werden kann und dort den Computer dazu veranlasst, folgende Schritte ausführen:
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind,
- Empfangen mindestens einer zweiten MRT-Aufnahme desselben Untersuchungsobjekts, wobei die mindestens eine zweite MRT-Aufnahme dieselbe Leber oder denselben Teil der Leber zeigt, wobei gesunde Leberzellen durch ein Kontrastmittel kontrastverstärkt dargestellt sind,
- Zuführen der empfangenen MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- Empfangen einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, als Ausgabe von dem Vorhersagemodel,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

Ein weiterer Gegenstand der vorliegenden Erfindung ist die Verwendung eines Kontrastmittels in einem MRT-Verfahren, wobei das MRT-Verfahren die folgenden Schritte umfasst:
- Applizieren des Kontrastmittels, wobei sich das Kontrastmittel in einer Leber eines Untersuchungsobjekts verteilt,
- Erzeugen mindestens einer ersten MRT-Aufnahme, wobei die mindestens eine erste MRT-Aufnahme die Leber oder einen Teil der Leber des Untersuchungsobjekts zeigt, wobei Blutgefäße in der Leber durch das Kontrastmittel kontrastverstärkt dargestellt sind,
- Erzeugen mindestens einer zweiten MRT-Aufnahme, wobei die mindestens eine zweite MRT-Aufnahme dieselbe Leber oder denselben Teil der Leber zeigt, wobei gesunde Leberzellen durch das Kontrastmittel kontrastverstärkt dargestellt sind,
- Zuführen der erzeugten MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- Empfangen einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, als Ausgabe von dem Vorhersagemodel,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

Ein weiterer Gegenstand ist ein Kontrastmittel zur Verwendung in einem MRT-Verfahren, wobei das MRT-Verfahren die folgenden Schritte umfasst:
- Applizieren des Kontrastmittels, wobei sich das Kontrastmittel in einer Leber eines Untersuchungsobjekts verteilt,
- Erzeugen mindestens einer ersten MRT-Aufnahme, wobei die mindestens eine erste MRT-Aufnahme die Leber oder einen Teil der Leber des Untersuchungsobjekts zeigt, wobei Blutgefäße in der Leber durch das Kontrastmittel kontrastverstärkt dargestellt sind,
- Erzeugen mindestens einer zweiten MRT-Aufnahme, wobei die mindestens eine zweite MRT-Aufnahme dieselbe Leber oder denselben Teil der Leber zeigt, wobei gesunde Leberzellen durch das Kontrastmittel kontrastverstärkt dargestellt sind,
- Zuführen der erzeugten MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- Empfangen einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, als Ausgabe von dem Vorhersagemodel,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

Ein weiterer Gegenstand ist ein Kit umfassend ein Kontrastmittel und ein erfindungsgemäßes Computerprogrammprodukt.

Die Erfindung wird nachstehend näher erläutert, ohne zwischen den Erfindungsgegenständen (Verfahren, System, Computerprogrammprodukt, Verwendung, Kontrastmittel zur Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt, Verwendung, Kontrastmittel zur Verwendung, Kit) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die vorliegende Erfindung erzeugt eine oder mehere künstliche MRT-Aufnahme(n) einer Leber oder eines Teils einer Leber eines Untersuchungsobjekts, wobei die eine oder die mehreren MRT-Aufnahme(n) die Leber oder den Teil der Leber ohne eine durch ein Kontrastmittel herbeigeführte Kontrastverstärkung zeigen. Die künstliche(n) MRT-Aufnahme(n) wird/werden auf der Basis von MRT-Aufnahmen erstellt, die alle mit einer durch ein Kontrastmittel herbeigeführten Kontrastverstärkung aufgenommen wurden. Die künstliche(n) MRT-Aufnahme(n) können mit Hilfe eines selbstlernenden Algorithmus erstellt werden und imitieren MRT-Aufnahme(n) von der Leber oder eines Teiles der Leber des Untersuchungsobjekts, welches nicht durch Applikation eines Kontrastmittels kontrastverstärkt wurde.

Das "Untersuchungsobjekt" ist üblicherweise ein Lebewesen, vorzugsweise ein Säugetier, ganz besonders bevorzugt ein Mensch.

Ein Teil des Untersuchungsobjekts wird einer kontrastverstärkten Magnetresonanztomographie-Untersuchung unterzogen. Der "Untersuchungsbereich", auch Aufnahmevolumen (engl.: *field of view,* FOV) genannt, stellt insbesondere ein Volumen dar, welches in den Magnetresonanzaufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden. Der Untersuchungsbereich umfasst zumindest einen Teil der Leber des Untersuchungsobjekts.

Der Untersuchungsbereich wird in ein Grundmagnetfeld eingebracht.

Dem Untersuchungsobjekt wird ein Kontrastmittel verabreicht, das sich in dem Untersuchungsbereich verteilt. Das Kontrastmittel wird vorzugsweise intravenös (zum Beispiel in eine Armvene) als Bolus, gewichtsadaptiert verabreicht.

Unter einem "Kontrastmittel" wird ein Stoff oder Stoffgemisch verstanden, dessen Anwesenheit in einer Magnetresonanzmessung zu einem veränderten Signal führt. Vorzugsweise führt das Kontrastmittel zu einer Verkürzung der T1-Relaxationszeit und/oder der T2-Relaxationszeit.

Vorzugsweise ist das Kontrastmittel ein hepatobiliäres Kontrastmittel wie beispielsweise Gd-EOB-DTPA oder Gd-BOPTA.

In einer besonders bevorzugten Ausführungsform handelt es sich bei dem Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

Der Untersuchungsbereich wird einem MRT-Verfahren unterzogen und dabei werden MRT-Aufnahmen erzeugt (gemessen), die den Untersuchungsbereich während der Untersuchungsphase zeigen.

Die gemessenen MRT-Aufnahmen können als zweidimensionale Bildaufnahmen vorliegen, die eine Schnittebene durch das Untersuchungsobjekt zeigen. Die gemessenen MRT-Aufnahmen können als Stapel zweidimensionaler Bildaufnahme vorliegen, wobei jede einzelne Bildaufnahme des Stapels eine andere Schnittebene zeigt. Die gemessenen MRT-Aufnahmen können als dreidimensionale Aufnahmen (3D-Aufnahmen) vorliegen. Aus Gründen der einfacheren Darstellung wird die Erfindung an einigen Stellen der vorliegenden Beschreibung anhand des Vorliegens von zweidimensionalen MRT-Aufnahmen erläutert, ohne die Erfindung jedoch auf zweidimensionale MRT-Aufnahmen beschränken zu wollen. Dem Fachmann ist klar, wie sich das jeweils Beschriebene auf Stapel zweidimensionaler Bildaufnahmen und auf 3D-Aufnahmen übertragen lässt (siehe hierzu z.B. M. Reisler, W. Semmler: *Magnetresonanztomographie,* Springer Verlag, 3. Auflage, 2002, ISBN: 978-3-642-63076-7).

Nach der intravenösen Applikation eines hepatobiliären Kontrastmittels in Form eines Bolus erreicht das Kontrastmittel die Leber zunächst über die Arterien. Diese sind in den entsprechenden MRT-Aufnahmen kontrastverstärkt dargestellt. Die Phase, in der die Leberarterien in MRT-Aufnahmen kontrastverstärkt dargestellt sind, wird als "arterielle Phase" bezeichnet. Diese Phase beginnt unmittelbar nach der Applikation des Kontrastmittels und dauert üblicherweise 15 bis 25 Sekunden.

Anschließend erreicht das Kontrastmittel die Leber über die Lebervenen. Während der Kontrast in den Leberarterien bereits abnimmt, erreicht der Kontrast in den Lebervenen ein Maximum. Die Phase, in der die Lebervenen in MRT-Aufnahmen kontrastverstärkt dargestellt sind, wird als "venöse Phase" bezeichnet. Diese Phase kann bereits während der arteriellen Phase beginnen und sich mit dieser überlagern. Üblicherweise startet diese Phase 20 bis 30 Sekunden nach der intravenösen Applikation und dauert üblicherweise 40 bis 60 Sekunden.

An die venöse Phase schließt sich die "Spätphase" an, in der der Kontrast in den Leberarterien weiter absinkt, der Kontrast in den Lebervenen ebenfalls absinkt und der Kontrast in den gesunden Leberzellen allmählich anstiegt. Diese Phase beginnt üblicherweise 70 bis 90 Sekunden nach der Applikation des Kontrastmittels und dauert üblicherweise 100 bis 120 Sekunden.

Die arterielle Phase, die venöse Phase und die Spätphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

Als "erste MRT-Aufnahme" wird in dieser Beschreibung eine MRT-Aufnahme bezeichnet, in der Blutgefäße erkennbar sind, die durch ein Kontrastmittel kontrastverstärkt dargestellt sind. Vorzugsweise handelt es sich bei der mindestens einen ersten MRT-Aufnahme um mindestens eine MRT-Aufnahme, die während der dynamischen Phase gemessen worden ist. Besonders bevorzugt handelt es sich um jeweils mindestens eine MRT-Aufnahme, die während der arteriellen Phase, der venösen Phase und/oder während der Spätphase gemessen worden ist. Ganz besonders bevorzugt handelt es sich um jeweils mindestens eine MRT-Aufnahme, die während der arteriellen, venösen und der Spätphase gemessen worden ist. Vorzugsweise handelt es sich bei der mindestens einen ersten MRT-Aufnahme um eine T1-gewichtete Darstellung.

Bei Verwendung eines paramagnetischen Kontrastmittels sind die Blutgefäße in der mindestens einen ersten MRT-Aufnahme aufgrund der Kontrastverstärkung durch eine hohe Signalintensität gekennzeichnet (signalreiche Darstellung). Diejenigen (zusammenhängenden) Strukturen innerhalb einer ersten MRT-Aufnahme, die eine Signalintensität innerhalb einer empirisch ermittelbaren Bandbreite aufweisen, lassen sich damit Blutgefäßen zuordnen. Damit liegt mit der mindestens einen ersten MRT-Aufnahme eine Information darüber vor, wo in den MRT-Aufnahmen Blutgefäße dargestellt sind bzw. welche Strukturen in den MRT-Aufnahmen auf Blutgefäße (Arterien und/oder Venen) zurückzuführen sind.

Als "zweite MRT-Aufnahme" wird in dieser Beschreibung eine MRT-Aufnahme bezeichnet, die den Untersuchungsbereich während der hepatobiliären Phase zeigt. Während der hepatobiliären Phase wird das gesunde Lebergewebe (Parenchym) kontrastverstärkt dargestellt. Diejenigen (zusammenhängenden) Strukturen innerhalb einer zweiten MRT-Aufnahme, die eine Signalintensität innerhalb einer empirisch ermittelbaren Bandbreite aufweisen, lassen sich damit gesunden Leberzellen zuordnen. Damit liegt mit der mindestens einen zweiten MRT-Aufnahme eine Information darüber vor, wo in den MRT-Aufnahmen gesunde Leberzellen dargestellt sind bzw. welche Strukturen in den MRT-Aufnahmen auf gesunde Leberzellen zurückzuführen sind. Vorzugsweise handelt es sich bei der mindestens einen zweiten MRT-Aufnahme um eine T1-gewichtete Darstellung.

Die MRT-Aufnahmen der dynamischen und der hepatobiliären Phase der Leber ziehen sich über eine vergleichsweise lange Zeitspanne hin. Über diese Zeitspanne sollten Bewegungen des Patienten vermieden werden, um Bewegungsartefakte in den MRT-Aufnahme zu minimieren. Die lang andauernde Bewegungseinschränkung kann für einen Patienten unangenehm sein. Daher etablieren sich mittlerweile verkürzte MRT-Aufnahmevorgehen, bei denen ein Kontrastmittels bereits eine gewisse Zeitspanne (d.h. 10 bis 20 Minuten) vor der MRT-Aufnahme dem Untersuchungsobjekt appliziert wird, um direkt MRT-Aufnahmen innerhalb der hepatobiliären Phase aufnehmen zu können. MRT-Aufnahmen der dynamischen Phase erfolgen dann im gleichen MRT-Aufnahmevorgang nach Applikation einer zweiten Dosis des Kontrastmittels. Im Vergleich zu einem konventionellen MRT-Aufnahmevorgang ist dadurch die Verweildauer eines Patienten bzw. eines Untersuchungsobjektes im MRT deutlich kürzer. Daher wird erfindungsgemäß bevorzugt die mindestens eine MRT-Aufnahme der Leber oder des Teils der Leber in der hepatobiliären Phase nach einer (ersten) Applikation eines ersten Kontrastmittels in das Untersuchungsobjekt aufgezeichnet und mindestens eine weitere MRT-Aufnahme derselben Leber oder des Teils derselben Leber in der dynamischen Phase nach Applikation eines zweiten Kontrastmittels oder einer zweiten Applikation des ersten Kontrastmittels in das gleiche Untersuchungsobjekt aufgezeichnet. Das erste Kontrastmittel ist dabei ein hepatobiliäres, paramagnetisches Kontrastmittel. Das zweite Kontrastmittel kann auch ein extrazelluläres, paramagnetisches Kontrastmittel sein.

Die "erste MRT-Aufnahme" und die "zweite MRT-Aufnahme" werden einem Vorhersagemodell zugeführt. Das Vorhersagemodell ist ein Modell, das konfiguriert ist, auf Basis der empfangenden MRT-Aufnahmen, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen.

Dabei bedeutet der Begriff "Vorhersage", dass die MRT-Aufnahmen, die die Leber oder einen Teil davon von einem Untersuchungsobjekt ohne durch ein Kontrastmittel herbeigeführte Kontrastverstärkung zeigen, unter Verwendung der MRT-Aufnahmen, die den gleichen Untersuchungsbereich mit durch ein Kontrastmittel herbeigeführter Kontrastverstärkung zeigen, berechnet werden.

Das Vorhersagemodell wurde vorzugsweise mit Hilfe eines selbstlernenden Algorithmus in einem überwachten maschinellen Lernen erstellt. Zum Lernen werden Trainingsdaten verwendet, die eine Vielzahl an MRT-Aufnahmen der dynamischen Phase und der hepatobiliären Phase der Leber oder eines Teils der Leber von einem Untersuchungsobjekt umfassen. Weiter wurden vorzugsweise auch Trainingsdaten verwendet, die von MRT-Aufnahmen der gleichen Leber bzw. Teils einer Leber des gleichen Untersuchungsobjekts erstellt wurden und bei denen keine Kontrastverstärkung vorlag, d.h. die ohne Applikation eines Kontrastmittels generiert wurden.

Der selbstlernende Algorithmus erzeugt beim maschinellen Lernen ein statistisches Modell, das auf den Trainingsdaten beruht. Das heißt, es werden nicht einfach die Beispiele auswendig gelernt, sondern der Algorithmus "erkennt" Muster und Gesetzmäßigkeiten in den Trainingsdaten. So kann der Algorithmus auch unbekannte Daten beurteilen. Validierungsdaten können verwendet werden, um die Güte der Beurteilung unbekannter Daten zu prüfen.

Das Trainieren des selbstlernenden Algorithmus erfolgt mittels überwachten Lernens (engl.: *supervised learning*)*,* d.h. dem Algorithmus werden nacheinander MRT-Aufnahmen mit Kontrastverstärkung in der dynamischen Phase und der hepatobiliären Phase präsentiert und es wird ihm mitgeteilt, welche nicht-kontrastverstärkten MRT-Aufnahmen mit diesen kontrastverstärkten MRT-Aufnahmen verbunden sind. Der Algorithmus lernt dann eine Beziehung zwischen den MRT-Aufnahmen mit Kontrastverstärkung und den MRT-Aufnahmen ohne Kontrastverstärkung, um für MRT-Aufnahmen mit Kontrastverstärkung ein oder mehrere MRT-Aufnahmen ohne Kontrastverstärkung vorherzusagen.

Selbstlernende Algorithmen, die mittels überwachten Lernens trainiert werden, sind vielfältig im Stand der Technik beschrieben (siehe z.B. C. Perez: Machine Learning Techniques: Supervised Learning and Classification, Amazon Digital Services LLC - Kdp Print Us, 2019, ISBN 1096996545, 9781096996545).

Vorzugsweise handelt es sich bei dem Vorhersagemodell um ein künstliches neuronales Netz.

Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von digitalen MRT-Aufnahmen als Eingangswerte. Normalerweise gibt es ein Eingangsneuron für jedes Pixel oder Voxel einer digitalen MRT-Aufnahme. Es können zusätzliche Eingangsneuronen für zusätzliche Eingangswerte (z.B. Informationen zum Untersuchungsbereich, zum Untersuchungsobjekt und/oder zu Bedingungen, die bei der Erzeugung der MRT-Aufnahmen herrschten) vorhanden sein.

In einem solchen Netzwerk dienen die Ausgangsneuronen dazu, für eine erste und eine zweite MRT-Aufnahme eine dritte künstliche MRT-Aufnahme zu generieren. Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN).

Ein Convolutional Neural Network ist in der Lage, Eingabedaten in Form einer Matrix zu verarbeiten. Dies ermöglicht es, als Matrix dargestellte digitale MRT-Aufnahmen (z.B. Breite x Höhe x Farbkanäle) als Eingabedaten zu verwenden. Ein normales neuronales Netz z.B. in Form eines Multi-Layer-Perceptrons (MLP) benötigt dagegen einen Vektor als Eingabe, d.h. um eine MRT-Aufnahme als Eingabe zu verwenden, müssten die Pixel oder Voxel der MRT-Aufnahme in einer langen Kette hintereinander ausgerollt werden. Dadurch sind normale neuronale Netze z.B. nicht in der Lage, Objekte in einer MRT-Aufnahme unabhängig von der Position des Objekts in der MRT-Aufnahme zu erkennen. Das gleiche Objekt an einer anderen Position in der MRT-Aufnahme hätte einen völlig anderen Eingabevektor.

Ein CNN besteht im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Bei der Analyse von Sequenzen (Abfolgen von MRT-Aufnahme) können Raum und Zeit als äquivalente Dimensionen behandelt und z.B. über 3D-Faltungen verarbeitet werden. Dies wurde in den Arbeiten von Baccouche et al. (Sequential Deep Learning for Human Action Recognition; International Workshop on Human Behavior Understanding, Springer 2011, pages 29-39) und Ji et al. (3D Convolutional Neural Networks for Human Action Recognition, IEEE Transactions on Pattern Analysis and Machine Intelligence, 35(1), 221-231) gezeigt. Ferner kann man verschiedene Netzwerke trainieren, die für Zeit und Raum verantwortlich sind, und schließlich die Merkmale verschmelzen, wie in Veröffentlichungen von Karpathy et al. (Large-scale Video Classification with Convolutional Neural Networks; Proceedings of the IEEE conference on Computer Vision and Pattern Recognition, 2014, pages 1725-1732) und Simonyan & Zisserman (Two-stream Convolutional Networks for Action Recognition in Videos; Advances in Neural Information Processing Systems, 2014, pages 568-576) beschrieben ist.

Rekurrente neuronale Netze (RNNs) sind eine Familie von so genannten Feedforward Neuronalen Netzen, die Rückkopplungsverbindungen zwischen Schichten enthalten. RNNs ermöglichen die Modellierung sequentieller Daten durch gemeinsame Nutzung von Parameterdaten über verschiedene Teile des neuronalen Netzwerks. Die Architektur für ein RNN enthält Zyklen. Die Zyklen repräsentieren den Einfluss eines gegenwärtigen Werts einer Variablen auf ihren eigenen Wert zu einem zukünftigen Zeitpunkt, da mindestens ein Teil der Ausgabedaten von der RNN als Rückkopplung zur Verarbeitung nachfolgender Eingaben in einer Sequenz verwendet wird.

Details sind dem Stand der Technik zu entnehmen (siehe z.B.: S. Khan et al.: A Guide to Convolutional Neural Networks for Computer Vision, Morgan & Claypool Publishers 2018, ISBN 1681730227, 9781681730226).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabevektoren auf gegebene Ausgabevektoren angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Beziehung der kontrastverstärkten MRT-Aufnahmen der dynamischen und hepatobiliären Phase und MRT-Aufnahmen ohne Kontrastverstärkung, die verwendet werden können, um eine oder mehrere MRT-Aufnahmen vorherzusagen, die einen Untersuchungsbereich ohne Kontrastverstärkung zeigen und die nur mittels kontrastverstärkten MRT-Aufnahmen des Untersuchungsbereichs berechnet werden.

Eine Kreuzvalidierungsmethode kann verwendet werden, um die Daten in Trainings- und Validierungsdatensätze aufzuteilen. Der Trainingsdatensatz wird beim Backpropagations-Training der Netzwerkgewichte verwendet. Der Validierungsdatensatz wird verwendet, um zu überprüfen, mit welcher Vorhersagegenauigkeit sich das trainierte Netzwerk auf unbekannte Mehrzahlen von MRT-Aufnahmen anwenden lässt.

Wie bereits angedeutet, können zum Trainieren, Validieren und Vorhersagen auch weitere Informationen zum Untersuchungsobjekt, zum Untersuchungsbereich und/oder zu Untersuchungsbedingungen verwendet werden.

Beispiele für Informationen zum Untersuchungsobjekt sind: Geschlecht, Alter, Gewicht, Größe, Anamnese, Art und Dauer und Menge bereits eingenommener Medikamente, Blutdruck, zentralvenöser Druck, Atemfrequenz, Serum, Albumin, Total Bilirubin, Blutzucker, Eisengehalt, Atemkapazität und dergleichen. Diese können z.B. auch einer Datenbank bzw. einer elektronischen Patientenakte entnommen werden.

Beispiele für Informationen zum Untersuchungsbereich sind: Vorerkrankungen, Operationen, Teilresektion, Lebertransplantation, Eisenleber, Fettleber und dergleichen.

Es ist denkbar, dass die empfangenen MRT-Aufnahmen einer retrospektiven Bewegungskorrektur unterzogen werden, bevor sie dem Vorhersagemodell zugeführt werden. Eine solche Bewegungskorrektur sorgt dafür, dass ein Pixel oder Voxel einer ersten MRT-Aufnahme denselben Untersuchungsbereich zeigt, wie das entsprechende Pixel oder Voxel einer zweiten, zeitlich nachgelagerten MRT-Aufnahme. Bewegungskorrekturverfahren sind im Stand der Technik beschrieben (siehe zum Beispiel: EP3118644, EP3322997, US20080317315, US20170269182, US20140062481, EP2626718).

Ein Gegenstand der vorliegenden Erfindung ist ein System, mit dem das erfindungsgemäße Verfahren ausgeführt werden kann.

Es ist denkbar, dass die genannten Einheiten Bestandteile eines einzigen Computersystems sind; es ist aber auch denkbar, dass die genannten Einheiten Bestandteile von mehreren separaten Computersystemen sind, die über ein Netzwerk miteinander verbunden sind, um Daten und/oder Steuersignale von einer Einheit zu einer anderen Einheit zu übermitteln.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem erfolgen über Eingabemittel wie beispielsweise eine Tastatur, eine Maus, ein Mikrofon, ein berührungsempfindliches Display und/oder dergleichen.

Das erfindungsgemäße System ist konfiguriert, mindestens eine erste MRT-Aufnahme mit Kontrastverstärkung der hepatobiliären Phase und mindestens eine zweite MRT-Aufnahme mit Kontrastverstärkung der dynamischen Phase zu empfangen und auf Basis dieser Daten und ggf. weiterer Daten eine oder mehrere MRT-Aufnahmen zu erzeugen (vorherzusagen, zu berechnen), die den Untersuchungsbereich d.h. die Leber oder Teile davon ohne Kontrastverstärkung zeigen.

Die Steuer- und Recheneinheit dient der Steuerung der Empfangseinheit, der Koordinierung der Daten- und Signalflüsse zwischen verschiedenen Einheiten und der Prozessierung und Erzeugung von MRT-Aufnahmen. Es ist denkbar, dass mehrere Steuer- und Recheneinheiten vorhanden sind.

Die Empfangseinheit dient zum Empfang von MRT-Aufnahmen. Die MRT-Aufnahmen können beispielsweise von einer Magnetresonanzanlage übermittelt werden oder aus einem Datenspeicher ausgelesen werden. Die Magnetresonanzanlage kann ein Bestandteil des erfindungsgemäßen Systems sein. Denkbar ist aber auch, dass das erfindungsgemäße System ein Bestandteil einer Magnetresonanzanlage ist.

Von der Empfangseinheit werden die mindestens eine erste MRT-Aufnahme und die mindestens eine zweite MRT-Aufnahme und ggf. weitere Daten an die Steuer- und Recheneinheit übermittelt.

Die Steuer- und Recheneinheit ist konfiguriert, anhand der MRT-Aufnahmen, die einen Untersuchungsbereich mit Kontrastverstärkung der dynamischen und der hepatobiliären Phase zeigen, eine oder mehrere MRT-Aufnahmen vorherzusagen, wobei die vorhergesagten MRT-Aufnahmen den Untersuchungsbereich ohne Kontrastverstärkung zeigen. Vorzugsweise kann in einen Arbeitsspeicher der Steuer- und Recheneinheit ein Vorhersagemodell geladen werden, mit dem die MRT-Aufnahmen ohne Kontrastverstärkung berechnet werden. Das Vorhersagemodell wurde vorzugsweise mit Hilfe eines selbstlernenden Algorithmus mittels überwachtem Lernen erzeugt (trainiert).

Über die Ausgabeeinheit kann die mindestens eine vorhergesagte MRT-Aufnahme angezeigt werden (zum Beispiel auf einem Monitor), ausgegeben werden (z.B. über einen Drucker) oder in einem Datenspeicher gespeichert werden.

Eine weitere Ausführungsform der Erfindung betrifft die Verwendung eines Kontrastmittels bzw. ein Kontrastmittel zur Verwendung in einem MRT-Verfahren, wobei das MRT-Verfahren die folgenden Schritte umfasst:
- Applizieren des Kontrastmittels, wobei sich das Kontrastmittel in einer Leber eines Untersuchungsobjekts verteilt,
- Erzeugen mindestens einer ersten MRT-Aufnahme, wobei die mindestens eine erste MRT-Aufnahme die Leber oder einen Teil der Leber des Untersuchungsobjekts zeigt, wobei Blutgefäße in der Leber durch das Kontrastmittel kontrastverstärkt dargestellt sind,
- Erzeugen mindestens einer zweiten MRT-Aufnahme, wobei die mindestens eine zweite MRT-Aufnahme dieselbe Leber oder denselben Teil der Leber zeigt, wobei gesunde Leberzellen durch das Kontrastmittel kontrastverstärkt dargestellt sind,
- Zuführen der erzeugten MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- Empfangen einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, als Ausgabe von dem Vorhersagemodel,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

In einer bevorzugten Variante wird die mindestens eine "zweite MRT-Aufnahme" nach einer (ersten) Applikation eines ersten Kontrastmittels in das Untersuchungsobjekt erzeugt und die mindestens eine "erste MRT-Aufnahme" nach einer zweiten Applikation des ersten Kontrastmittels oder einer Applikation eines zweiten Kontrastmittels in das gleiche Untersuchungsobjekt erzeugt. D.h., dass die oben definierte "zweite MRT-Aufnahme" zeitlich vor der oben definierten "ersten MRT-Aufnahme" erzeugt wird.

Die Erfindung wird nachstehend anhand von Figuren näher erläutert, ohne die Erfindung auf die in den Figuren gezeigten Merkmale oder Merkmalskombinationen beschränken zu wollen.

Es zeigen:

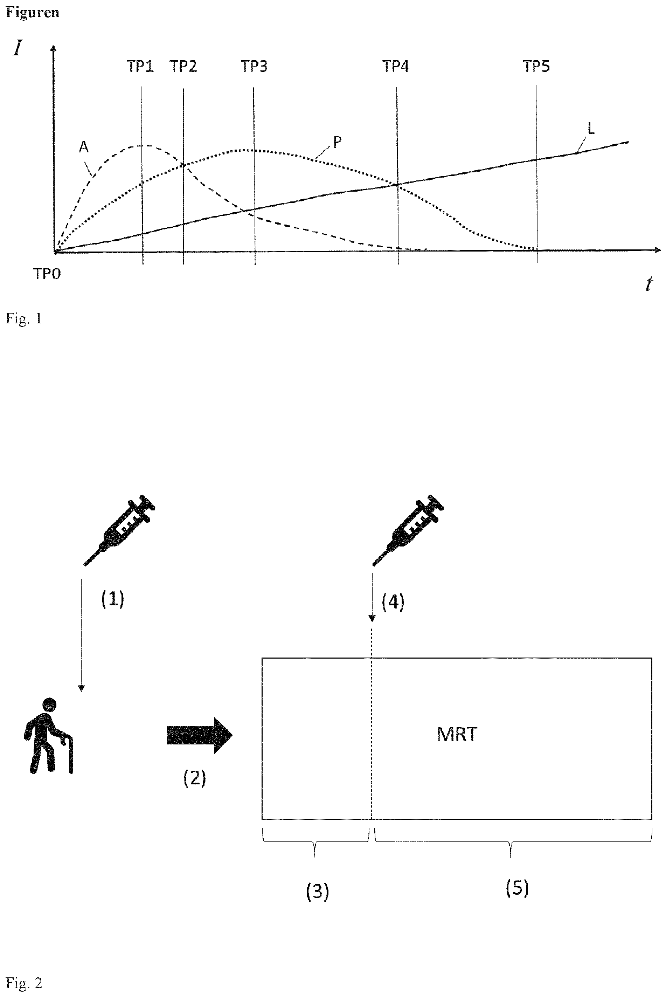

Figur 1 zeigt schematisch den zeitlichen Verlauf der Konzentrationen von Kontrastmittel in den Leberarterien (A), den Lebervenen (P) und den Leberzellen (L). Die Konzentrationen sind in Form der Signalintensitäten I in den genannten Arealen (Leberarterien, Lebervenen, Leberzellen) bei der Magnetresonanzmessung als Funktion der Zeit t dargestellt. Bei einer intravenösen Bolusinjektion steigt die Konzentration des Kontrastmittels in den Leberarterien (A) als erstes an (gestrichelte Kurve). Die Konzentration durchläuft ein Maximum und sinkt dann ab. Die Konzentration in den Lebervenen (P) steigt langsamer an als in den Leberarterien und erreicht ihr Maximum später (gepunktete Kurve). Die Konzentration des Kontrastmittels in den Leberzellen (L) steigt langsam an (durchgezogene Kurve) und erreicht ihr Maximum erst zu einem sehr viel späteren Zeitpunkt (in der Figur 1 nicht dargestellt). Es lassen sich einige charakteristische Zeitpunkte definieren: Zum Zeitpunkt TP0 wird Kontrastmittel intravenös als Bolus appliziert. Zum Zeitpunkt TP1 erreicht die Konzentration (die Signalintensität) des Kontrastmittels in den Leberarterien ihr Maximum. Zum Zeitpunkt TP2 schneiden sich die Kurven der Signalintensitäten bei den Leberarterien und den Lebervenen. Zum Zeitpunkt TP3 durchläuft die Konzentration (die Signalintensität) des Kontrastmittels in den Lebervenen ihr Maximum. Zum Zeitpunkt TP4 schneiden sich die Kurven der Signalintensitäten bei den Lebervenen und den Leberzellen. Zum Zeitpunkt TP5 sind die Konzentrationen in den Leberarterien und den Lebervenen auf ein Niveau gesunken, bei dem sie keine messbare Kontrastverstärkung mehr verursachen.
Figur 2 zeigt schematisch ein Beispiel für ein verkürztes MRT-Aufnahmevorgehen. Bei einem verkürzten MRT-Aufnahmevorgehen wird zuerst ein Kontrastmittel appliziert (1). Das Untersuchungsobjekt wird nach einem gewissen Wartezeitraum z.B. 10 bis 20 Minuten (2) dem MRT zugeführt. Danach wird der MRT-Vorgang gestartet und zuerst ein MRT der Leber bzw. eines Teils davon in der hepatobiliären Phase durchgeführt (3). Danach wird dem Untersuchungsobjekt eine weitere intravenöse Bolusinjektion (4) appliziert und direkt anschließend ein MRT der Leber bzw. eines Teils davon in der dynamischen Phase durchgeführt.

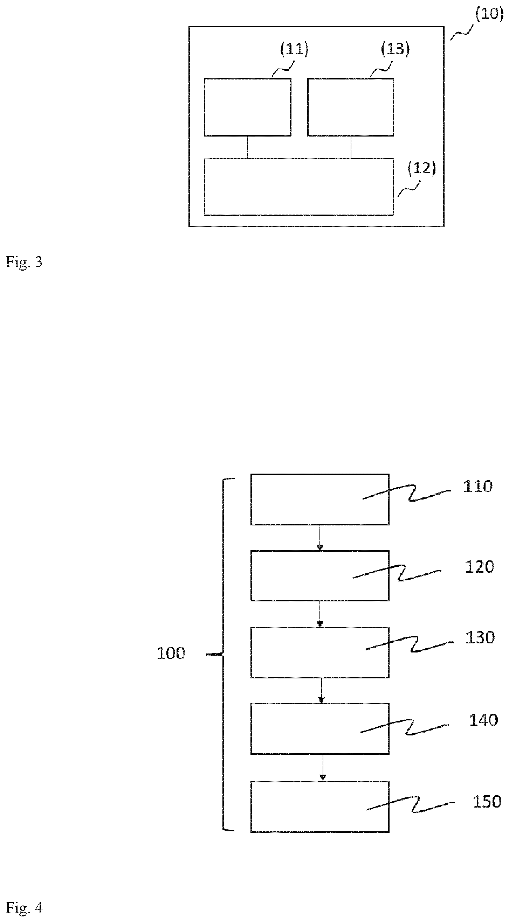

Figur 3 zeigt schematisch eine bevorzugte Ausführungsform des erfindungsgemäßen Systems. Das System (10) umfasst eine Empfangseinheit (11), eine Steuer- und Recheneinheit (12) und eine Ausgabeeinheit (13).

Die Steuer- und Recheneinheit (12) ist konfiguriert, die Empfangseinheit (11) zu veranlassen, mindestens eine erste MRT-Aufnahme eines Untersuchungsobjekts zu empfangen, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind.

Die Steuer- und Recheneinheit (12) ist ferner konfiguriert, die Empfangseinheit (11) zu veranlassen, mindestens eine zweite MRT-Aufnahme eines Untersuchungsobjekts zu empfangen, wobei die mindestens eine zweite MRT-Aufnahme dieselbe Leber oder denselben Teil der Leber zeigt, wobei gesunde Leberzellen durch ein Kontrastmittel kontrastverstärkt dargestellt sind.

Die Steuer- und Recheneinheit (12) ist ferner konfiguriert, anhand der empfangenen MRT-Aufnahmen eine oder mehrere MRT-Aufnahmen vorherzusagen, wobei die eine oder die mehreren vorhergesagten MRT-Aufnahmen die Leber oder einen Teil der Leber des Untersuchungsobjekts ohne eine durch ein Kontrastmittel herbeigeführte Kontrastverstärkung zeigen.

Die Steuer- und Recheneinheit (12) ist ferner konfiguriert, die Ausgabeeinheit (13) zu veranlassen, die mindestens eine vorhergesagte MRT-Aufnahme anzuzeigen, auszugeben oder in einem Datenspeicher zu speichern.

Figur 4 zeigt schematisch und beispielhaft eine Ausführungsform des erfindungsgemäßen Verfahrens.

Das Verfahren (100) umfasst die Schritte:
- (110) Empfangen mindestens einer ersten MRT-Aufnahme eines Untersuchungsobjekts, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind,
- (120) Empfangen mindestens einer zweiten MRT-Aufnahme desselben Untersuchungsobjekts, wobei die mindestens eine zweite MRT-Aufnahme dieselbe Leber oder denselben Teil der Leber zeigt, wobei gesunde Leberzellen durch ein Kontrastmittel kontrastverstärkt dargestellt sind,
- (130) Zuführen der empfangenen MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- (140) Empfangen einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, von dem Vorhersagemodell,
- (150) Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

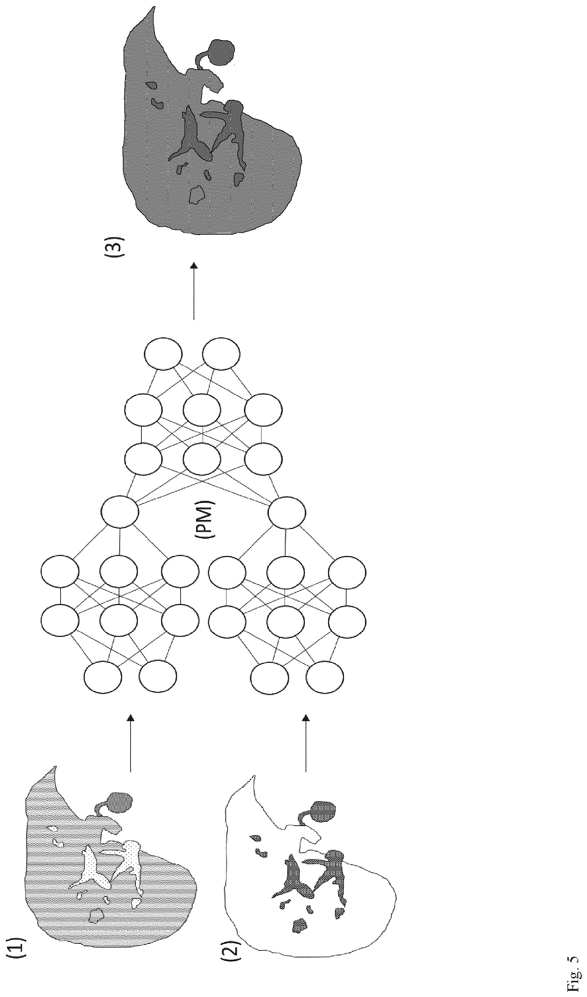

Figur 5 zeigt beispielhaft und schematisch eine weitere Ausführungsform der vorliegenden Erfindung. Es wird eine erste MRT-Aufnahme (1) bereitgestellt, wobei die erste MRT-Aufnahme eine Leber oder einen Teil einer Leber eines Untersuchungsobjekts zeigt, wobei Blutgefäße in der Leber durch ein Kontrastmittel kontrastverstärkt (signalverstärkt) dargestellt sind.

Es wird eine zweite MRT-Aufnahme (2) bereitgestellt, wobei die zweite MRT-Aufnahme dieselbe Leber oder denselben Teil der Leber zeigt wie die erste MRT-Aufnahme, wobei das gesunde Lebergewebe (Parenchym) durch ein Kontrastmittel kontrastverstärkt (signalverstärkt) dargestellt ist.

Die erste MRT-Aufnahme (1) und die zweite MRT-Aufnahme (2) werden einem Vorhersagemodell (PM) zugeführt.

Das Vorhersagemodell (PM) ist konfiguriert, auf Basis der ersten MRT-Aufnahme (1) und der zweiten MRT-Aufnahme (2) eine dritte MRT-Aufnahme (3) zu erzeugen, die eine MRT-Aufnahme ohne eine durch ein Kontrastmittel hervorgerufene Kontrastverstärkung zeigt.

Das Vorhersagemodell wurde vorzugsweise mit Hilfe eines selbstlernenden Algorithmus in einem überwachten maschinellen Lernen mit einem Trainingsdatensatz erstellt. Der Trainingsdatensatz umfasst eine Vielzahl an ersten MRT-Aufnahmen, zweiten MRT-Aufnahmen und den dazugehörigen dritten MRT-Aufnahmen, wobei die dritten MRT-Aufnahmen tatsächlich aufgenommen wurden z.B. vor Applikation eines ersten intravenösen Bolus des Kontrastmittels.

Der selbstlernende Algorithmus erzeugt beim maschinellen Lernen ein statistisches Modell, das auf den Trainingsdaten beruht. Das heißt, es werden nicht einfach die Beispiele auswendig gelernt, sondern der Algorithmus "erkennt" Muster und Gesetzmäßigkeiten in den Trainingsdaten. So kann der Algorithmus auch unbekannte Daten beurteilen. Validierungsdaten können verwendet werden, um die Güte der Beurteilung unbekannter Daten zu prüfen.

Das Trainieren des selbstlernenden Algorithmus erfolgt mittels überwachten Lernens (engl.: supervised learning), d.h. dem Algorithmus werden erste und zweite MRT-Aufnahmen präsentiert und es wird ihm mitgeteilt, welche dritten MRT-Aufnahmen mit den jeweiligen ersten und zweiten MRT-Aufnahmen verbunden sind. Der Algorithmus lernt dann eine Beziehung zwischen den MRT-Aufnahmen, um für unbekannte erste und zweite MRT-Aufnahmen dritte MRT-Aufnahmen vorherzusagen (zu berechnen).

Selbstlernende Algorithmen, die mittels überwachten Lernens trainiert werden, sind vielfältig im Stand der Technik beschrieben (siehe z.B. C. Perez: Machine Learning Techniques: Supervised Learning and Classification, Amazon Digital Services LLC - Kdp Print Us, 2019, ISBN 1096996545, 9781096996545).

Vorzugsweise handelt es sich bei dem Vorhersagemodell um ein künstliches neuronales Netz.

Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von digitalen MRT-Aufnahmen als Eingangswerte. Normalerweise gibt es ein Eingangsneuron für jedes Pixel oder Voxel einer digitalen MRT-Aufnahme. Es können zusätzliche Eingangsneuronen für zusätzliche Eingangswerte (z.B. Informationen zum Untersuchungsbereich, zum Untersuchungsobjekt und/oder zu Bedingungen, die bei der Erzeugung der MRT-Aufnahmen herrschten) vorhanden sein.

In einem solchen Netzwerk dienen die Ausgangsneuronen dazu, für eine erste und eine zweite MRT-Aufnahme eine dritte MRT-Aufnahme zu generieren.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN).

Ein Convolutional Neural Network ist in der Lage, Eingabedaten in Form einer Matrix zu verarbeiten. Dies ermöglicht es, als Matrix dargestellte digitale MRT-Aufnahmen (z.B. Breite x Höhe x Farbkanäle) als Eingabedaten zu verwenden. Ein normales neuronales Netz z.B. in Form eines Multi-Layer-Perceptrons (MLP) benötigt dagegen einen Vektor als Eingabe, d.h. um eine MRT-Aufnahme als Eingabe zu verwenden, müssten die Pixel oder Voxel der MRT-Aufnahme in einer langen Kette hintereinander ausgerollt werden. Dadurch sind normale neuronale Netze z.B. nicht in der Lage, Objekte in einer MRT-Aufnahme unabhängig von der Position des Objekts in der MRT-Aufnahme zu erkennen. Das gleiche Objekt an einer anderen Position in der MRT-Aufnahme hätte einen völlig anderen Eingabevektor.

Ein CNN besteht im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Details sind dem Stand der Technik zu entnehmen (siehe z.B.: S. Khan et al.: A Guide to Convolutional Neural Networks for Computer Vision, Morgan & Claypool Publishers 2018, ISBN 1681730227, 9781681730226).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabevektoren auf gegebene Ausgabevektoren angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Beziehung der kontrastverstärkten MRT-Aufnahmen der dynamischen und hepatobiliären Phase und MRT-Aufnahmen ohne Kontrastverstärkung, die verwendet werden können, um eine oder mehrere MRT-Aufnahmen vorherzusagen, die einen Untersuchungsbereich ohne Kontrastverstärkung zeigen und die nur mittels kontrastverstärkten MRT-Aufnahmen des gleichen Untersuchungsbereichs berechnet werden.

Eine Kreuzvalidierungsmethode kann verwendet werden, um die Daten in Trainings- und Validierungsdatensätze aufzuteilen. Der Trainingsdatensatz wird beim Backpropagations-Training der Netzwerkgewichte verwendet. Der Validierungsdatensatz wird verwendet, um zu überprüfen, mit welcher Vorhersagegenauigkeit sich das trainierte Netzwerk auf unbekannte Mehrzahlen von MRT-Aufnahmen anwenden lässt.

## Patentansprüche

1. Verfahren (100) umfassend die Schritte
- Empfangen (110) mindestens einer ersten MRT-Aufnahme eines Untersuchungsobjekts, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind,
- Empfangen (120) mindestens einer zweiten MRT-Aufnahme desselben Untersuchungsobjekts, wobei die mindestens eine zweite MRT-Aufnahme dieselbe Leber oder denselben Teil der Leber zeigt, wobei gesunde Leberzellen durch ein Kontrastmittel kontrastverstärkt dargestellt sind,
- Zuführen (130) der empfangenen MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- Empfangen (140) einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, von dem Vorhersagemodell
- Anzeigen und/oder Ausgeben (150) der einen oder der mehreren vorhergesagten MRT-Aufnahmen und/oder Speichern der einen oder der mehreren vorhergesagten MRT-Aufnahmen in einem Datenspeicher.

2. Verfahren gemäß Anspruch 1, wobei es sich bei der mindestens einen ersten MRT-Aufnahme um eine T1-gewichtete Darstellung der Leber oder des Teils der Leber in der dynamischen Phase nach Applikation eines hepatobiliären, paramagnetischen Kontrastmittels handelt.

3. Verfahren gemäß Anspruch 2, wobei es sich bei der mindestens einen ersten MRT-Aufnahme um MRT-Aufnahmen handelt, die
(i) die Leber oder einen Teil der Leber des Untersuchungsobjekts während der arteriellen Phase zeigen,
(ii) dieselbe Leber oder denselben Teil der Leber des gleichen Untersuchungsobjekts während der venösen Phase zeigen, und
(iii) dieselbe Leber oder denselben Teil der Leber des gleichen Untersuchungsobjekts während der Spätphase zeigen.

4. Verfahren gemäß einem der Ansprüche 1 bis 3, wobei es sich bei der mindestens einen zweiten MRT-Aufnahme um eine T1-gewichtete Darstellung der Leber oder des Teils der Leber in der hepatobiliären Phase nach Applikation eines hepatobiliären, paramagnetischen Kontrastmittels oder eines extrazellulären, paramagnetischen Kontrastmittels handelt.

5. Verfahren gemäß einem der Ansprüche 2 bis 4, wobei die mindestens eine zweite MRT-Aufnahme mit einer T1-gewichtete Darstellung der Leber oder des Teils der Leber in der hepatobiliären Phase nach einer ersten Applikation eines hepatobiliären, paramagnetischen Kontrastmittels in das Untersuchungsobjekt aufgezeichnet wird, und die mindestens eine erste MRT-Aufnahme mit einer T1-gewichtete Darstellung derselben Leber oder des Teils derselben Leber in der dynamischen Phase nach einer zweiten Applikation des hepatobiliären, paramagnetischen Kontrastmittels oder eines extrazellulären, paramagnetischen Kontrastmittels in das gleiche Untersuchungsobjekt aufgezeichnet wird.

6. Verfahren gemäß einem der Ansprüche 1 bis 5, wobei es sich bei dem Kontrastmittel um einen Stoff oder ein Stoffgemisch mit der Gadoxetsäure oder einem Salz der Gadoxetsäure als kontrastverstärkenden Wirkstoff, bevorzugt um das Dinatriumsalz der Gadoxetsäure handelt.

7. Verfahren gemäß einem der vorherigen Ansprüche, wobei es sich beim Untersuchungsobjekt um ein Säugetier, vorzugsweise um einen Menschen handelt.

8. Verfahren gemäß einem der vorherigen Ansprüche, wobei es sich bei dem Vorhersagemodell um ein künstliches neuronales Netz handelt.

9. System (10) umfassend
• eine Empfangseinheit (11),
• eine Steuer- und Recheneinheit (12) und
• eine Ausgabeeinheit (13),
- wobei die Steuer- und Recheneinheit konfiguriert ist, die Empfangseinheit zu veranlassen, mindestens eine erste MRT-Aufnahme eines Untersuchungsobjekts zu empfangen, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind,
- wobei die Steuer- und Recheneinheit konfiguriert ist, die Empfangseinheit zu veranlassen, mindestens eine zweite MRT-Aufnahme eines Untersuchungsobjekts zu empfangen, wobei die mindestens eine zweite MRT-Aufnahme dieselbe Leber oder denselben Teil der Leber zeigt, wobei gesunde Leberzellen durch ein Kontrastmittel kontrastverstärkt dargestellt sind,
- wobei die Steuer- und Recheneinheit konfiguriert ist, anhand der empfangenen MRT-Aufnahmen eine oder mehrere MRT-Aufnahmen mit einem Vorhersagemodell vorherzusagen, wobei die eine oder die mehreren vorhergesagten MRT-Aufnahmen die Leber oder einen Teil der Leber des Untersuchungsobjekts ohne eine durch ein Kontrastmittel herbeigeführte Kontrastverstärkung zeigen und wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- wobei die Steuer- und Recheneinheit konfiguriert ist, die Ausgabeeinheit zu veranlassen, die eine oder die mehreren vorhergesagte MRT-Aufnahmen anzuzeigen, auszugeben oder in einem Datenspeicher zu speichern.

10. Computerprogrammprodukt umfassend ein Computerprogramm, das in einen Arbeitsspeicher eines Computers geladen werden kann und dort den Computer dazu veranlasst, folgende Schritte ausführen:
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind,
- Empfangen mindestens einer zweiten MRT-Aufnahme desselben Untersuchungsobjekts, wobei die mindestens eine zweite MRT-Aufnahme dieselbe Leber oder denselben Teil der Leber zeigt, wobei gesunde Leberzellen durch ein Kontrastmittel kontrastverstärkt dargestellt sind,
- Zuführen der empfangenen MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- Empfangen einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, als Ausgabe von dem Vorhersagemodel,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

11. Computerprogrammprodukt gemäß Anspruch 10, wobei das Computerprogramm den Computer dazu veranlasst, einen oder mehrere der in den Ansprüchen 1 bis 7 aufgeführten Schritte auszuführen.

12. Verwendung eines Kontrastmittels in einem MRT-Verfahren, wobei das MRT-Verfahren die folgenden Schritte umfasst:
- Applizieren des Kontrastmittels, wobei sich das Kontrastmittel in einer Leber eines Untersuchungsobjekts verteilt,
- Erzeugen mindestens einer ersten MRT-Aufnahme, wobei die mindestens eine erste MRT-Aufnahme die Leber oder einen Teil der Leber des Untersuchungsobjekts zeigt, wobei Blutgefäße in der Leber durch das Kontrastmittel kontrastverstärkt dargestellt sind,
- Erzeugen mindestens einer zweiten MRT-Aufnahme, wobei die mindestens eine zweite MRT-Aufnahme dieselbe Leber oder denselben Teil der Leber zeigt, wobei gesunde Leberzellen durch das Kontrastmittel kontrastverstärkt dargestellt sind,
- Zuführen der erzeugten MRT-Aufnahmen einem Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts zeigen und in denen die Blutgefäße in der Leber durch ein Kontrastmittel kontrastverstärkt dargestellt sind, und von MRT-Aufnahmen derselben Leber oder demselben Teil der Leber des gleichen Untersuchungsobjekts, in denen gesunde Leberzellen durch ein Kontrastmittel kontrastverstärkt dargestellt sind, eine oder mehrere MRT-Aufnahmen vorherzusagen, die die Leber oder einen Teil der Leber des Untersuchungsobjekts ohne eine durch ein Kontrastmittel herbeigeführte Kontrastverstärkung zeigen,
- Empfangen einer oder mehrerer vorhergesagter MRT-Aufnahmen, die die Leber oder einen Teil der Leber des Untersuchungsobjekts ohne eine durch ein Kontrastmittel herbeigeführte Kontrastverstärkung zeigen, als Ausgabe von dem Vorhersagemodel,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

13. Kit umfassend ein Kontrastmittel gemäß Anspruch 12 und ein Computerprogrammprodukt gemäß Anspruch 10 oder 11.

## Claims

1. Method (100) comprising the steps of
- receiving (110) at least one first MRI image of an examination object, the at least one first MRI image showing a liver or part of a liver of the examination object, blood vessels in the liver being depicted with contrast enhancement as a result of a contrast agent,
- receiving (120) at least one second MRI image of the same examination object, the at least one second MRI image showing the same liver or the same part of the liver, healthy liver cells being depicted with contrast enhancement as a result of a contrast agent,
- feeding (130) the received MRI images to a prediction model, the prediction model having been trained by means of supervised learning to predict, on the basis of MRI images which show a liver or part of a liver of an examination object and in which the blood vessels in the liver are depicted with contrast enhancement as a result of a contrast agent and on the basis of MRI images of the same liver or the same part of the liver of the same examination object in which healthy liver cells are depicted with contrast enhancement as a result of a contrast agent, one or more MRI images showing the liver or part of the liver of the examination object without a contrast enhancement caused by a contrast agent,
- receiving (140) from the prediction model one or more predicted MRI images showing the liver or part of the liver of the examination object without a contrast enhancement caused by a contrast agent,
- displaying and/or outputting (150) the one or more predicted MRI images and/or storing the one or more predicted MRI images in a data memory.

2. Method according to Claim 1, wherein the at least one first MRI image is a T1-weighted depiction of the liver or the part of the liver in the dynamic phase after administration of a hepatobiliary, paramagnetic contrast agent.

3. Method according to Claim 2, wherein the at least one first MRI image comprises MRI images which
(i) show the liver or part of the liver of the examination object during the arterial phase,
(ii) show the same liver or the same part of the liver of the same examination object during the venous phase, and
(iii) show the same liver or the same part of the liver of the same examination object during the late phase.

4. Method according to any of Claims 1 to 3, wherein the at least one second MRI image is a T1-weighted depiction of the liver or the part of the liver in the hepatobiliary phase after administration of a hepatobiliary, paramagnetic contrast agent or an extracellular, paramagnetic contrast agent.

5. Method according to any of Claims 2 to 4, wherein the at least one second MRI image with a T1-weighted depiction of the liver or the part of the liver in the hepatobiliary phase is recorded after a first administration of a hepatobiliary, paramagnetic contrast agent into the examination object, and the at least one first MRI image with a T1-weighted depiction of the same liver or the part of the same liver in the dynamic phase is recorded after a second administration of the hepatobiliary, paramagnetic contrast agent or an extracellular, paramagnetic contrast agent into the same examination object.

6. Method according to any of Claims 1 to 5, wherein the contrast agent is a substance or a substance mixture with gadoxetic acid or a gadoxetic acid salt as contrast-enhancing active substance, preferably the disodium salt of gadoxetic acid.

7. Method according to any of the preceding claims, wherein the examination object is a mammal, preferably a human.

8. Method according to any of the preceding claims, wherein the prediction model is an artificial neural network.

9. System (10) comprising
• a receiving unit (11),
• a control and calculation unit (12) and
• an output unit (13),
- the control and calculation unit being configured to cause the receiving unit to receive at least one first MRI image of an examination object, the at least one first MRI image showing a liver or part of a liver of the examination object, blood vessels in the liver being depicted with contrast enhancement as a result of a contrast agent,
- the control and calculation unit being configured to cause the receiving unit to receive at least one second MRI image of an examination object, the at least one second MRI image showing the same liver or the same part of the liver, healthy liver cells being depicted with contrast enhancement as a result of a contrast agent,
- the control and calculation unit being configured to predict one or more MRI images on the basis of the received MRI images by means of a prediction model, the one or more predicted MRI images showing the liver or part of the liver of the examination object without a contrast enhancement caused by a contrast agent and the prediction model having been trained by means of supervised learning to predict, on the basis of MRI images which show a liver or part of a liver of an examination object and in which the blood vessels in the liver are depicted with contrast enhancement as a result of a contrast agent and on the basis of MRI images of the same liver or the same part of the liver of the same examination object in which healthy liver cells are depicted with contrast enhancement as a result of a contrast agent, one or more MRI images showing the liver or part of the liver of the examination object without a contrast enhancement caused by a contrast agent,
- the control and calculation unit being configured to cause the output unit to display the one or more predicted MRI images, to output them or to store them in a data memory.

10. Computer program product comprising a computer program that can be loaded into a working memory of a computer, where it causes the computer to execute the following steps:
- receiving at least one first MRI image of an examination object, the at least one first MRI image showing a liver or part of a liver of the examination object, blood vessels in the liver being depicted with contrast enhancement as a result of a contrast agent,
- receiving at least one second MRI image of the same examination object, the at least one second MRI image showing the same liver or the same part of the liver, healthy liver cells being depicted with contrast enhancement as a result of a contrast agent,
- feeding the received MRI images to a prediction model, the prediction model having been trained by means of supervised learning to predict, on the basis of MRI images which show a liver or part of a liver of an examination object and in which the blood vessels in the liver are depicted with contrast enhancement as a result of a contrast agent and on the basis of MRI images of the same liver or the same part of the liver of the same examination object in which healthy liver cells are depicted with contrast enhancement as a result of a contrast agent, one or more MRI images showing the liver or part of the liver of the examination object without a contrast enhancement caused by a contrast agent,
- receiving as output from the prediction model one or more predicted MRI images showing the liver or part of the liver of the examination object without a contrast enhancement caused by a contrast agent,
- displaying and/or outputting the one or more predicted MRI images and/or storing the one or more predicted MRI images in a data memory.

11. Computer program product according to Claim 10, wherein the computer program causes the computer to execute one or more of the steps listed in Claims 1 to 7.

12. Use of a contrast agent in an MRI method, the MRI method comprising the following steps:
- administering the contrast agent, the contrast agent spreading in a liver of an examination object,
- generating at least one first MRI image, the at least one first MRI image showing the liver or part of the liver of the examination object, blood vessels in the liver being depicted with contrast enhancement as a result of the contrast agent,
- generating at least one second MRI image, the at least one second MRI image showing the same liver or the same part of the liver, healthy liver cells being depicted with contrast enhancement as a result of the contrast agent,
- feeding the generated MRI images to a prediction model, the prediction model having been trained by means of supervised learning to predict, on the basis of MRI images which show a liver or part of a liver of an examination object and in which the blood vessels in the liver are depicted with contrast enhancement as a result of a contrast agent and on the basis of MRI images of the same liver or the same part of the liver of the same examination object in which healthy liver cells are depicted with contrast enhancement as a result of a contrast agent, one or more MRI images showing the liver or part of the liver of the examination object without a contrast enhancement caused by a contrast agent,
- receiving as output from the prediction model one or more predicted MRI images showing the liver or part of the liver of the examination object without a contrast enhancement caused by a contrast agent,
- displaying and/or outputting the one or more predicted MRI images and/or storing the one or more predicted MRI images in a data memory.

13. Kit comprising a contrast agent according to Claim 12 and a computer program product according to Claim 10 or 11.

## Revendications

1. Procédé (100) comprenant les étapes
- réception (110) d'au moins une première image IRM d'un objet d'examen, ladite au moins une première image IRM montrant un foie ou une partie d'un foie de l'objet d'examen, les vaisseaux sanguins dans le foie étant représentés avec un contraste accentué par un agent de contraste,
- réception (120) d'au moins une deuxième image IRM du même objet d'examen, ladite au moins une deuxième image IRM montrant le même foie ou la même partie du foie, les cellules hépatiques saines étant représentées avec un contraste accentué par un agent de contraste,
- envoi (130) des images IRM reçues à un modèle de prédiction, le modèle de prédiction ayant été entraîné au moyen d'apprentissage supervisé, à l'aide d'images IRM qui montrent un foie ou une partie d'un foie d'un objet d'examen et dans lesquelles les vaisseaux sanguins dans le foie sont représentés avec un contraste accentué par un agent de contraste, et d'images IRM du même foie ou de la même partie du foie du même objet d'examen, dans lesquelles les cellules hépatiques saines sont représentées avec un contraste accentué par un agent de contraste, pour prédire une ou plusieurs images IRM qui montrent le foie ou une partie du foie de l'objet d'examen sans une accentuation de contraste provoquée par un agent de contraste,
- réception (140) depuis le modèle de prédiction d'une ou de plusieurs images IRM prédites qui montrent le foie ou une partie du foie de l'objet d'examen sans une accentuation de contraste provoquée par un agent de contraste,
- affichage et/ou sortie (150) de ladite une ou desdites plusieurs images IRM prédites et/ou mémorisation de ladite ou desdites plusieurs images IRM prédites dans une mémoire de données.

2. Procédé selon la revendication 1, dans lequel pour ce qui est de ladite au moins une première image IRM il s'agit d'une représentation pondérée en T1 du foie ou de la partie du foie dans la phase dynamique après application d'un agent de contraste paramagnétique hépatobiliaire.

3. Procédé selon la revendication 2, dans lequel pour ce qui est de ladite au moins une première image IRM il s'agit d'images IRM qui
(i) montrent le foie ou une partie du foie de l'objet d'examen pendant la phase artérielle,
(ii) montrent le même foie ou la même partie du foie du même objet d'examen pendant la phase veineuse, et
(iii) montrent le même foie ou la même partie du foie du même objet d'examen pendant la phase tardive, et

4. Procédé selon l'une quelconque des revendications 1 à 3, dans lequel ladite au moins une deuxième image IRM est une représentation pondérée en T1 du foie ou de la partie du foie dans la phase hépatobiliaire après application d'un agent de contraste paramagnétique hépatobiliaire ou d'un agent de contraste paramagnétique extracellulaire.

5. Procédé selon l'une quelconque des revendications 2 à 4, dans lequel ladite au moins une deuxième image IRM comportant une représentation pondérée en T1 du foie ou de la partie du foie dans la phase hépatobiliaire est enregistrée après une première application d'un agent de contraste paramagnétique hépatobiliaire dans l'objet d'examen, et ladite au moins une première image IRM est enregistrée avec une représentation pondérée en T1 du même foie ou de la partie du même foie dans la phase dynamique après une deuxième application du produit de contraste paramagnétique hépatobiliaire ou d'un produit de contraste paramagnétique extracellulaire dans le même objet d'examen.

6. Procédé selon l'une quelconque des revendications 1 à 5, dans lequel pour ce qui est de l'agent de contraste il s'agit d'une substance ou d'un mélange de substances contenant de l'acide gadoxétique ou un sel de l'acide gadoxétique en tant qu'agent d'accentuation de contraste, de préférence du sel disodique de l'acide gadoxétique.

7. Procédé selon l'une quelconque des revendications précédentes, dans lequel pour ce qui est de l'objet d'examen il s'agit d'un mammifère, de préférence d'un être humain.

8. Procédé selon l'une quelconque des revendications précédentes, dans lequel pour ce qui est du modèle de prédiction il s'agit d'un réseau neuronal artificiel.

9. Système (10) comprenant
• • une unité de réception (11),
• • une unité de commande et calcul (12), et
• • une unité de sortie (13),
- dans lequel l'unité de commande et calcul est configurée pour amener l'unité de réception à recevoir au moins une première image IRM d'un objet d'examen, ladite au moins une première image IRM montrant un foie ou une partie d'un foie de l'objet d'examen, les vaisseaux sanguins du foie étant représentés dans le foie avec un contraste accentué par un agent de contraste,
- dans lequel l'unité de commande et calcul est configurée pour amener l'unité de réception à recevoir au moins une deuxième image IRM d'un objet d'examen, ladite au moins une deuxième image IRM montrant le même foie ou la même partie du foie, les cellules hépatiques saines étant représentées avec un contraste accentué par un agent de contraste,
- dans lequel l'unité de commande et calcul est configurée pour prédire avec un modèle de prédiction, à l'aide des images IRM reçues, une ou plusieurs images IRM, ladite une ou lesdites plusieurs images IRM prédites montrant le foie ou une partie du foie de l'objet d'examen sans une accentuation de contraste provoquée par un agent de contraste et le modèle de prédiction ayant été entraîné au moyen d'apprentissage supervisé, à l'aide d'images IRM qui montrent un foie ou une partie d'un foie d'un objet d'examen et dans lesquelles les vaisseaux sanguins dans le foie sont représentés avec un contraste accentué par un agent de contraste, et d'images IRM du même foie ou de la même partie du foie du même objet d'examen, dans lesquelles les cellules hépatiques saines sont représentées avec un contraste accentué par un agent de contraste, pour prédire une ou plusieurs images IRM qui montrent le foie ou une partie du foie de l'objet d'examen sans une accentuation de contraste provoquée par un agent de contraste,
- dans lequel l'unité de commande et calcul est configurée pour amener l'unité de sortie à afficher, à sortir ou à mémoriser dans une mémoire de données ladite une ou lesdites plusieurs images IRM prédites.

10. Produit programme informatique, comprenant un programme informatique qui peut être chargé dans une mémoire vive d'un ordinateur et qui y amène l'ordinateur à exécuter les étapes suivantes :
- réception d'au moins une première image IRM d'un objet d'examen, ladite au moins une première image IRM montrant un foie ou une partie d'un foie de l'objet d'examen, les vaisseaux sanguins dans le foie étant représentés avec un contraste accentué par un agent de contraste,
- réception d'au moins une deuxième image IRM du même objet d'examen, ladite au moins une deuxième image IRM montrant le même foie ou la même partie du foie, les cellules hépatiques saines étant représentées avec un contraste accentué par un agent de contraste,
- envoi des images IRM reçues à un modèle de prédiction, le modèle de prédiction ayant été entraîné au moyen d'apprentissage supervisé, à l'aide d'images IRM qui montrent un foie ou une partie d'un foie d'un objet d'examen et dans lesquelles les vaisseaux sanguins dans le foie sont représentés avec un contraste accentué par un agent de contraste, et d'images IRM du même foie ou de la même partie du foie du même objet d'examen, dans lesquelles les cellules hépatiques saines sont représentées avec un contraste accentué par un agent de contraste, pour prédire une ou plusieurs images IRM qui montrent le foie ou une partie du foie de l'objet d'examen sans une accentuation de contraste provoquée par un agent de contraste,
- réception d'une ou de plusieurs images IRM prédites qui montrent le foie ou une partie du foie de l'objet d'examen sans une accentuation de contraste provoquée par un agent de contraste, en tant que sortie du modèle de prédiction,
- affichage et/ou transmission de ladite une ou desdites plusieurs images IRM prédites et/ou mémorisation de ladite ou desdites plusieurs images IRM prédites dans une mémoire de données.

11. Produit programme informatique selon la revendication 10, dans lequel le programme informatique amène l'ordinateur à exécuter une ou plusieurs des étapes indiquées dans les revendications 1 à 7.

12. Utilisation d'un agent de contraste dans un procédé d'IRM, le procédé d'IRM comprenant les étapes suivantes :
- application de l'agent de contraste, l'agent de contraste se répartissant dans un foie d'un objet d'examen,
- production d'au moins une première image IRM, ladite au moins une première image IRM montrant le foie ou une partie du foie de l'objet d'examen, les vaisseaux sanguins dans le foie étant représentés avec un contraste accentué par l'agent de contraste,
- production d'au moins une deuxième image IRM, ladite au moins une deuxième image IRM montrant le même foie ou la même partie du foie, les cellules hépatiques saines étant représentées avec un contraste accentué par l'agent de contraste,
- envoi des images IRM produites à un modèle de prédiction, le modèle de prédiction ayant été entraîné au moyen d'apprentissage supervisé, à l'aide d'images IRM qui montrent un foie ou une partie d'un foie d'un objet d'examen et dans lesquelles les vaisseaux sanguins dans le foie sont représentés avec un contraste accentué par un agent de contraste, et d'images IRM du même foie ou de la même partie du foie du même objet d'examen, dans lesquelles les cellules hépatiques saines sont représentées avec un contraste accentué par un agent de contraste, pour prédire une ou plusieurs images IRM qui montrent le foie ou une partie du foie de l'objet d'examen sans une accentuation de contraste provoquée par un agent de contraste,
- réception d'une ou de plusieurs images IRM prédites qui montrent le foie ou une partie du foie de l'objet d'examen sans une accentuation de contraste provoquée par un agent de contraste, en tant que sortie du modèle de prédiction,
- affichage et/ou transmission de ladite une ou desdites plusieurs images IRM prédites et/ou mémorisation de ladite ou desdites plusieurs images IRM prédites dans une mémoire de données.

13. Kit comprenant un agent de contraste selon la revendication 12 et un produit programme informatique selon la revendication 10 ou 11.
